**Europäisches Patentamt**

**European Patent Office**

**Office européen des brevets**

(19)

(11) Publication number: **0 084 157**
A2

(12) # EUROPEAN PATENT APPLICATION

(21) Application number: **82111896.5**

(22) Date of filing: **22.12.82**

(51) Int. Cl.³: **A 61 K 7/48**

(30) Priority: **28.12.81 JP 210810/81**

(43) Date of publication of application:
**27.07.83 Bulletin 83/30**

(84) Designated Contracting States:
**DE FR GB IT**

(71) Applicant: **Sansho Seiyaku Co., Ltd.**
**1-6 Oaza Tsutsui**
**Ohnojo-shi Fukuoka-ken(JP)**

(71) Applicant: **Kyoei Kagaku Kogyo Co., Ltd.**
**12-19 Minamihorie 1-chome Nishi-ku**
**Osaka-shi Osaka-fu(JP)**

(72) Inventor: **Izumi, Tokio**
**12-3 Otoganedai 2-chome**
**Ohnojo-shi Fukuoka-ken(JP)**

(74) Representative: **Patentanwälte Müller-Boré, Deufel,**
**Schön, Hertel, Lewald, Otto**
**Postfach 86 07 20 Siebertstrasse 4**
**D-8000 München 86(DE)**

(54) **Cosmetics.**

(57) A cosmetic composition for chappy skin comprising as blend ingredient an extract produced by mincing human umbilical cord or cockscomb, digesting the minced product with pronase and then filtering the resultant product.

EP 0 084 157 A2

Croydon Printing Company Ltd

- 2 -

3. DETAILED DESCRIPTION OF THE INVENTION

This invention relates to a cosmetic composition and, more particularly, to a cosmetic composition which has water retention characteristics, excellent wrinkle-smoothing effect and adaptability for chappy skin.

Chappy skin is one of the dermal disorders most commonly seen and hitherto various studies have been made on this chappy skin. In particular, chappy skin has been recently found to be caused from moisture deficiency in stratum corneum, i.e. skin epithelium. If stratum corneum may contain 10 to 20 % moisture, skin may be kept under soft condition, but it becomes fragile and dry if moisture is not more than 10 %. Further, it was reported that such moisture retention in stratum corneum can be accomplished by the presence of a certain hydrophilic substance (Proceedings of Scientific Section, Toilet Goods Association, No.23, 1923, May, 1955, by Irvin H. Blank) and it was also known in the art that this hydrophilic substance is a substance called "Natural Moisturriging Factor" (hereinafter referred to frequently as NMF).

Thereafter, it has been proposed that a variety of mucopolysaccharides might be applied as cosmetics with the expectation that mucopolysaccharides are also present in stratum corneum, in addition to said NMF, and may place a certain role for moisture retention. For example, Japanese Patent Publication No. 500/1958 discloses that a mixture of

- 3 -

hyaluronic acid, mucoitin sulfate and charonin sulfuric acid is applied as cell-activating cosmetics and Japanese Patent Publication No. 2700/1957 discloses cell-activating cosmetics containing chondroitin sulfuric acid.

However, expected effects could not have been achieved by addition of such mucopolysaccharides solely. Then, the present applicants made studies on substance that would act with the aforesaid NMF for moisture retention in stratum corneum and enhance its effect and, as a result, found that a substance derived from such raw material as umbilical cord, pigskin, cockscomb, vitreous body in eye-ball of animals and fishes through proteolytic enzyme treatment is extremely effective for moisture retention in stratum cornerum and also that cosmetics containing as a main ingredient said substance can exhibit prominent effects on human chappy skin (See, Japanese Patent Publication No. 11178/1976).

The present inventor has made further extensive studies with regard to the previous invention on treatment procedures thereof, especially on the raw materials, enzymes to be applied and treating conditions for more advantageous availability of the active substance in cosmetics according to the previous invention noted above and eventually completed the present invention.

This invention is directed to cosmetics which comprise an extract that is produced by mincing umbilical cord or

- 4 -

cockscomb, digesting the minced product with pronase and then filtering the resultant product.

Umbilical cord and cockscomb, which may be employed as raw material in this invention, may be utilizable in the form of umbilical cord itself as separated from human placenta or cockscomb itself as cut from cock or in the form of a defatted product therefrom with a suitable organic solvent, e.g. acetone and the like.

Pronase is the proteolytic enzyme that is produced by Streptomyces griseus and can rupture peptide bonds of various protein substrates almost indiscriminately. Its enzymatic activity is stable at a pH range of 5-9 and its optimum pH is 7-8. As commercially available pronase, there may be mentioned Pronase E, P, AS and AF (available from Kaken Chemical Co., Ltd., Japan): titre is 70,000 PUK/g for Pronase E, 45,000 PUK/g for Pronase P, 15,000 PUK/g for Pronase AS and 15,000 PUK/g for Pronase AF.

There may be suitably employed any of said pronases for this invention, but it is preferable, for example, for Pronase P to employ the enzyme at a titre corresponding to 0.005-0.2 titre per one of the raw material.

The process for preparing the extract, i.e., the blend ingredient for the present cosmetics is to mince the above-mentioned umbilical cord, cockscomb and like material and add thereto pronase, one of proteolytic enzymes. More specifically, the raw material is previously swollen with water thoroughly, minced by means of a suitable mincing machine and then pronase is added to the minced product.. Alternatively, the raw material may be minced and then swollen thoroughly by dipping it into water. Namely, the so swollen liquid product may be homogenized and then pronase is added thereto to effect enzymatic treatment. Treatment with pronase is usually conducted at a pH range between about 6 and about 8, but it is preferable in this instance to conduct the treatment at an approximately neutral pH range in view of working conditions and others. An amount of pronase to be added is usually at a ratio of about 0.005 to 0.2 to 1 (weight) of the raw·material. Pronase treatment is usually completed at a temperature of about 50 to 60°C in 1 to 10 hours.

This pronase treatment can accomplish degradation of a complex of mucopolysaccharide with protein in tissue of the raw material umbilical cord and cockscomb to afford such pronase degradation products as various mucopolysaccharides, proteins, peptides, amino acids and the like. By the use of pronase for degradation of umbilical cord and cockscomb as stated above, there can be given the following advantages, as compared with the instances using trypsin or pepsine:

(1) Enzyme activity of pronase can be exerted even under heating to about 50 to 60°C because of its heat resistance and, consequently, contamination with other

- 6 -

bacteria can be prevented during the degradation process;

(2) pronase has a high degradation activity and accomplish random cleavage of peptide bonds in protein;

(3) pronase is easily available as the enzyme is producible by a microorganism; and

(4) pronase has a wide activity range of pH 6 to 8 and hence treatment can be effected at a neutral range, which leads to satisfactory working conditions, and, furthermore, treating activity of pronase remains unchanged even with more or less pH changes in the liquid to be treated.

Thereafter, insoluble materials can be removed from the pronase-treated liquid thus obtained by means of any suitable filtration means to give the blend ingredient for the present cosmetics.

This blend ingredient is a liquid comprising mainly acidic polysaccharides such as hyaluronic acid, chondroitin sulfuric acid and the like, together with proteins and amino acid admixed therewith. For instance, the acidic polysaccharide in the pronase-treated liquid from umbilical cord may usually comprise 50 to 70 % by weight of hyaluronic acid and 50 to 30 % by weight of chondroitin sulfuric acid, while the polysaccharide derived from cockscomb may comprise 60 to 80 % by weight of hyaluronic acid and 20 to 40 % by weight of chondroitin sulfuric acid.

When the pronase-treated liquid derived from umbilical cord or cockscomb or a mixture thereof is applied to skin in the form of a cosmetic, it can enhance water retention characteristics in stratum corneum, stabilize and activate connective tissues, dialate peripheral blood vessel to improve blood circulation and further exert functions to activate intermediary metabolism for acceleration of nourishment and excretion of waste products. As a consequence, a chappy skin can be prevented and treated and also an excellent wrinkle-smoothing effect can be accomplished.

Water retention effect was tested with the present active ingredient, in comparison with mucopolysacchride, namely hyaluronic acid and chondoloitin sulfuric acid alone or in combination therewith. The test results are summarized in the following Table 1, wherein the results with water as a control are also shown.

### Test Method

A weighing bottle with an inner diameter of 4 cm and a height of 6 cm was fitted with a cap having a copper wire hook at the lower part thereof and then weighed. Cut epithelia from rats (each having an area of 2 $cm^2$ and a thickness of 0.3 mm) were dipped into the umbilical cord extract according to this invention, a 2 % aqueous solution of hyaluronic acid, a 2 % aqueous solution of chondroitin sulfuric acid, a 2 % aqueous solution of a mixture of hyaluronic acid with chondroitin sulfuric acid (7 : 3 (wieght)) and water, respectively, and subsequently each epithelium was hanged with the hook in the bottle. Another weighing bottle containing 20 ml of conc. sulfuric acid was maintained at 23°C, the cap in the previously prepared bottle was removed and fitted in this bottle and then epithelia were dried until a constant weight was reached. Then, the conc. sulfuric acid was replaced with dilute sufuric acid having a specific gravity of 1.473, thereby producing an environment of a relative humidity of 23 % which is similar to the external circumstance. The epithelia as dried to a constant weight as stated above were hanged within said environment and then subjected to moisture absorption until a constant weight was reached over 48 to 72 hours. According to this test method, a moisture amount absorbed into the epithelium was measured under a condition equilibrated to the open air having a certain relative humidity by increasing an amount of dilute sulfuric acid.

- 9 -

Table 1

Bound moisture (mg/100 mg of dried epithelium weight)

| Relative humidity (%) | Active ingredient extract of this invention | Hyaluronic acid | Chondoroitin sulfuric acid | Hyaluronic acid + Chondoroitin sulfuric acid | Control ($H_2O$) |
|---|---|---|---|---|---|
| 30 | 11 | 4 | 3 | 8 | 3 |
| 40 | 13 | 6 | 4 | 7 | 3 |
| 50 | 15 | 9 | 6 | 10 | 5 |
| 60 | 17 | 10 | 7 | 11 | 8 |
| 70 | 25 | 17 | 13 | 19 | 13 |
| 80 | 38 | 29 | 27 | 30 | 26 |
| 90 | 67 | 51 | 47 | 55 | 45 |

As apparent from the above results, the umbilical cord extract, the active ingredient according to this invention, is superior in water retention characteristics to mucopolysaccharide alone or a mixture thereof.

Where the above extract of umbilical cord or cockscomb is to be employed as cosmetics, the extract, as it stands or after treated, e.g., decolored and further purified, may be blended into a cosmetic base on vehicle to prepare a cosmetic composition.

- 10 -

An amount of the blend ingredient of this invention to be blended may be suitably selected depending upon the form of a cosmetic composition and the skin condition to be applied, but a favourable effect may be in general shown at about 10 to 3 %.

As examples of the cosmetic forms which the present blend ingredient may be applied to, there may be preferably mentioned a milky lotion, a lotion, a cream and other forms which may be easily applied to skin.

For preparing such cosmetic forms, one may generally utilize any cosmetic bases or vehicles and procedures commonly employed in the art for such purposes.

This invention will be more fully illustrated by way of the non-limiting Preparations and Examples as set forth below.

Preparation 1

To 1 kg of fresh umbilical cord after washing with water was added a three times volume of water and the resulting mixture was minced by a mixer, pronase was added thereto and then treatment was effected over 8 hours while heating to 60$^{o}$C. Thereafter, the resulting treated liquid was filtered to give a clear solution. The solution was concentrated at 60$^{o}$C under reduced pressure to a total volume of one-fourth and further suction-filtered to afford 1 kg of the desired extract.

- 11 -

Preparation 2

To 1 kg of fresh cockscomb after washing with water was added a five times volume of water and the resulting mixture was minced by a mixer, pronase was added thereto and then treatment was effected over 8 hours while heating to 60°C. Thereafter, the resulting liquid was treated in the same manner as in Preparation 1 to afford 1 to 2 kg of the desired cockscomb extract.

Example 1

The following formulations (a) and (b) were heated to 80°C, respectively, and then they were admixed and stirred. When the resulting mixture was allowed to cool to 50°C, the following component (c) was added thereto and stirred to produce a milky lotion.

| | | |
|---|---|---|
| (a) | Cetanol | 3.5 g |
| | Liquid paraffin | 5.0 g |
| | Squalane | 4.0 g |
| | dl-$\alpha$-Tocopherol | 0.05 g |
| | Butyl p-oxybenzoate | 0.1 g |
| | Glycerol monostearate | 0.7 g |
| | Polyoxyethylene cetyl ether | 2.0 g |
| (b) | Carboxyvinyl polymer | 0.2 g |

Blend ingredient obtained

in Preparation 1              0.5 g

Potassium carbonate     0.07 g

Methyl p-oxybenzoate     0.5 g

Purified water to make 100 g.


(c)  Perfumery           0.1 g


Example 2

The following formulations (a) and (b) were heated to 80°C, respectively, and then they were admixed and stirred. When the resulting mixture was allowed to cool to 50°C, the following component (c) was added thereto and stirred to produce a cream.

(a)  Stearic acid           3.0 g

      Beeswax              4.0 g

      Liquid paraffin      10.0 g

      Squalane            8.0 g

      Glycerol monostearate    1.5 g

      Polyoxyethylene hardened

      castor oil           3.0 g

      Polyoxyethylene stearyl ether  1.2 g

      dl-$\alpha$-Tocopherol      0.05 g

      Butyl p-oxybenzoate     0.1 g

- 13 -

(b)  Glycerol                                    2 g

     Ethyl p-oxybenzoate                          0.1 g

     Blend ingredient obtained

     in Preparation 2                             2 g

     Purified water to make 100 g.


(c)  Perfumery                                    0.2 g


Example 3

The following components were admixed and stirred to produce a lotion.

     Ethanol                                      10 g

     Glycerol                                     2.0 g

     Citric acid                                  0.5 g

     Blend ingredient obtained

     in Preparation 2                             5.0 g

     Methyl p-oxybenzoate                         0.2 g

     Perfumery                                    0.05 g

     Purified water to make 100 g.


Example 4

The following components were admixed to produce a pack.

     Ethanol                                      5.0 g

     Glycerol                                     5.0 g

Mixture of blends obtained

in Preparations 1 and 2      10.0 g

Methyl p-oxybenzoate      0.2 g

Carboxyvinyl polymer      1.5 g

Potassium carbonate      0.6 g

Perfumery      0.1 g

Purified water to make 100 g.

The following experiment is given in order to show toxicity of the present blend.

Experiment

Male dd-strain mice weighing 18 to 20 g were used in this experiment. The blend ingredient obtained in the above Preparation 1 was orally administered via oral sound to animals. Acute toxicity of the blend ingredient was examined through observation over 72 hours after the administration. The results are summarized in the following Table.

Table

|  | Administered volume (ml/20 g body weight) | Number of animals used | Number of dead animals |
|---|---|---|---|
| Blend ingredient obtained in Preparation 1 | 0.1 | 10 | 0 |
|  | 0.5 | 10 | 0 |
|  | 1.0 | 10 | 0 |
| Control (Physiological saline) | 1.0 | 10 | 0 |

As apparent from the above results, no one of anima died after 72 hours even at the maximum dose of 1.0 ml/20 body weight which may be regarded as physical limitati and, moreover, any toxic actions were not observed in tl test groups similarly to the control group with physiolog cal saline even when symptoms were observed within one ho after the administration.

What is claimed is:

1.  A cosmetic composition for chappy skin comprising conventional cosmetic bases or vehicles characterized in that an extract is blended therein, said extract being produced by mincing human umbilical cord or cockscomb, digesting the minced product with pronase and then filtering the resultant product.

2.  A cosmetic composition as claimed in claim 1, in which said pronase is used at 0.005 to 0.2 per 1 of said raw material.

3.  A cosmetic composition as claimed in claim 1, in which said composition is in the form of a milky lotion, a cream, a lotion or a pack.

4.  A cosmetic composition as claimed in claim 1, in which said extract is blended therein at 10 to 3 %.